# EUROPEAN PATENT SPECIFICATION

(11) **EP 3 322 456 B1**
(45) Date of publication and mention of the grant of the patent: **04.11.2020**
(21) Application number: 16751330.8
(22) Date of filing: 06.07.2016
(51) Int. Cl.: A61M 1/10, A61M 1/12

(54) **A NEW CIRCULATORY SUPPORT DEVICE USING THE ENDOGENOUS RESPIRATORY OR MUSCULAR ENERGY: A NOVEL NATIVE ENERGY INVERTER.**
NEUE KREISLAUFUNTERSTÜTZUNGSVORRICHTUNG MIT NUTZUNG DER ENDOGENEN ATEM- ODER MUSKELENERGIE: NEUARTIGER INVERTER VON NATIVER ENERGIE
NOUVEAU DISPOSITIF D'ASSISTANCE CIRCULATOIRE UTILISANT L'ÉNERGIE MUSCULAIRE OU RESPIRATOIRE ENDOGÈNE : NOUVEL INVERSEUR D'ÉNERGIE NATIVE

(30) Priority: 14.07.2015 GR 20150100310
(43) Date of publication of application: 23.05.2018
(73) Proprietor: Stefanadis, Christodoulos, 15452 Athens (GR)
(72) Inventor: Stefanadis, Christodoulos, 15452 Athens (GR)
(74) Representative: Kilimiris, Constantinos
(86) International application number: PCT/GR2016/000030
(87) International publication number: WO 2017/009672

(56) References cited:
- US-A- 3 518 702
- US-A- 4 078 267
- US-A- 5 813 410
- US-A- 6 086 526

## Description

### Technical field and Background of the invention

Temporary circulatory support devices (see e.g. US3518702) which are implanted in patients with end-stage heart failure, usually as a bridge therapy to heart transplantation, depend on external energy (mechanical, electric, etc.), are demanding in management and have high cost.

### Summary of the invention

The present device refers to a novel circulatory support device which functions using the native energy from the respiratory system of the patient himself. Thus, the main advantage of this device is that it does not depend on external source of energy, as it works as an energy inverter, leading to transformation of one form of energy (respiratory) to another (cardiac). The invention is defined in independent claim 1.

### Detailed explanation of preferred embodiments

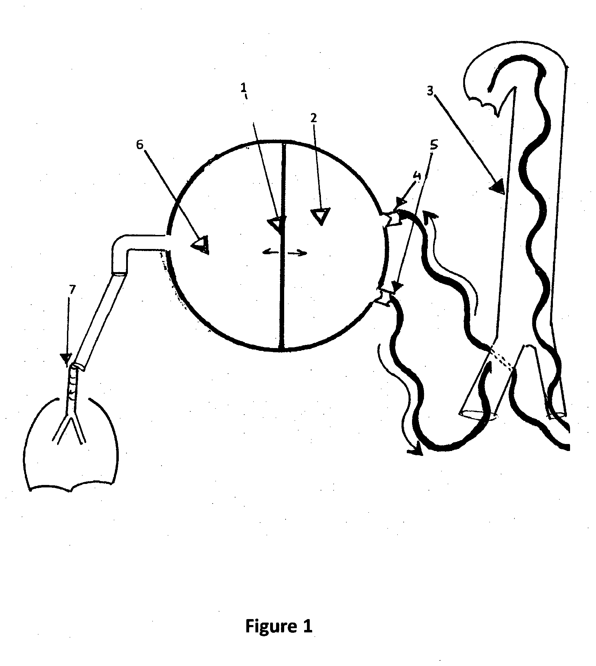

The present device is a pump with two chambers separated by an elastic membrane (1). ***In*** ***Figure 1******,*** the one chamber (2) is connected to the aorta (3) or left ventricle through two, one-way valves: an inlet (4) and an outlet valve (5). The second chamber (6) is connected to the upper respiratory trunk eg. trachea (7) and is supplied by air of variable pressure.

By the movement of the elastic membrane, the blood is pumped to the circulation during the inspiratory phase.

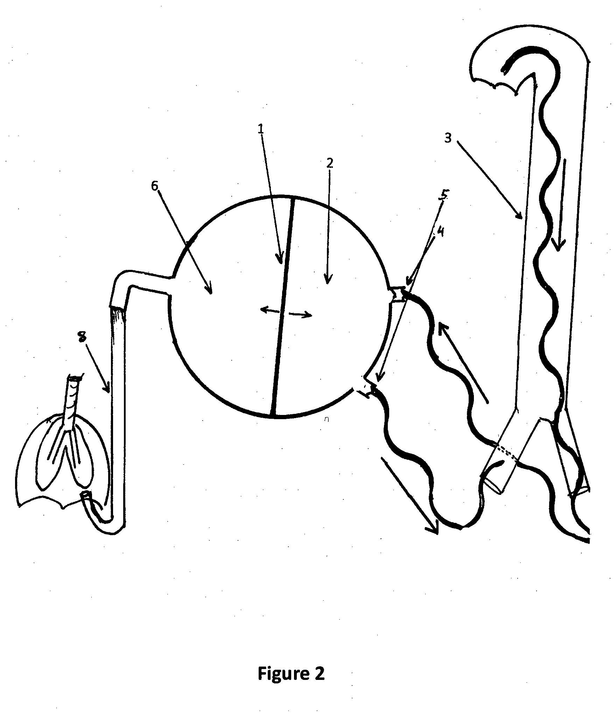

In ***Figure 2******,*** the one chamber (2) is connected to the aorta (3) or left ventricle through two, one-way valves: an inlet (4) and an outlet valve (5).

The other chamber is connected to the pleural cavity through a tube (8). The two chambers are separated by an elastic membrane (1). The movement of the elastic membrane is achieved by the pressure variations within the pleural cavity (intrathoracic pressure).

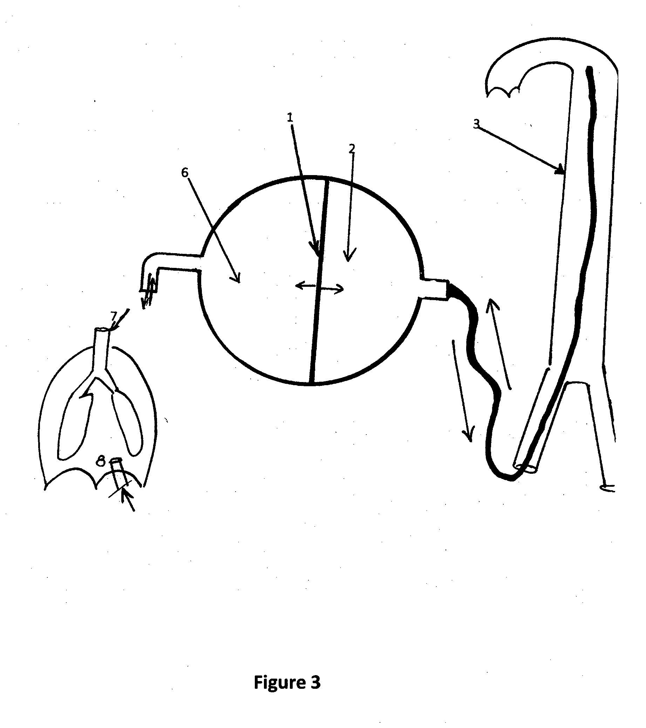

In ***Figure 3******,*** a modified version of the novel device is presented: the one chamber (2) is connected to the aorta (3) or left ventricle without the need of a valve. The other chamber is connected either to upper respiratory
tract (7) or the pleural cavity through a tube (8). The elastic membrane (1) is moved by the respiratory movements and the blood is, periodically, pumped to the circulation.

## Claims

1. A new, circulatory support device being adapted to use energy from the respiratory system of the patient himself, the support device comprising a pump which consists of a first chamber (2) adapted to be connected to the blood circuit (3) and a second chamber (6) adapted to be connected to the respiratory system (7) wherein blood is circulated in the blood circuit when air pressure variations induced during respiratory cycle cause pressure variations in the second chamber (6).

2. A new, circulatory support device, according to claim 1, which may work with energy derived from the muscular movements of the patient himself.

3. A new, circulatory support device, according to claim 1, comprising mechanical ventilators connected to the support device, so that circulatory support is promoted in intubated patients with cardiogenic shock.

4. Circulatory support device according to claim 1 wherein the first chamber is adapted to be connected to aorta or the left ventricle through two one-way valves and the second chamber is adapted to be connected to the upper respiratory trunk, therefore being configured to be supplied by air of variable pressure, wherein the first and second chambers are separated by an elastic membrane, in such a way that the movement of the elastic membrane due to the respiratory cycle forces blood to be pumped.

5. Circulatory support device according to claim 1 wherein the first chamber is adapted to be connected to adapted to be aorta or the left ventricle through two one-way valves and the second chamber is adapted to be connected to the pleural cavity through a tube, therefore being configured to be supplied by air of variable pressure, wherein the first and second chambers are separated by an elastic membrane, in such a way that the movement of the elastic membrane due to the respiratory cycle forces blood to be pumped.

6. Circulatory support device according to claim 1 wherein the first chamber (2) is adapted to be connected to the aorta (3) or left ventricle without the need of a valve, the second chamber is adapted to be connected either to upper respiratory tract (7) or the pleural cavity through a tube (8), and an elastic membrane (1) separates first and second chambers in such a way that the elastic membrane is moved by the respiratory movements, thus causing the blood to be periodically pumped to the circulation.

7. Circulatory support device according to claim 6, wherein the connection between the first chamber (2) and the aorta or left ventricle is made by means of a conduit.

## Patentansprüche

1. Neue Kreislaufunterstützungsvorrichtung, die geeignet ist, Energie aus dem Atmungssystem des Patienten selbst zu nutzen, wobei die Unterstützungsvorrichtung eine Pumpe umfasst, die besteht aus einer ersten Kammer (2), die an den Blutkreislauf (3) anschließbar ist, und einer zweiten Kammer (6), die an das Atmungssystem (7) anschließbar ist, wobei Blut im Blutkreislauf zirkuliert wird, wenn es infolge von während des Atemzyklus auftretenden Luftdruckschwankungen zu Druckschwankungen in der zweiten Kammer (6) kommt.

2. Neue Kreislaufunterstützungsvorrichtung nach Anspruch 1, die mit Energie arbeiten kann, welche aus den Muskelbewegungen des Patienten selbst gewonnen wird.

3. Neue Kreislaufunterstützungsvorrichtung nach Anspruch 1, umfassend mechanische Beatmungsgeräte, die mit der Unterstützungsvorrichtung verbunden sind, um die Kreislaufunterstützung bei intubierten Patienten mit kardiogenem Schock zu fördern.

4. Kreislaufunterstützungsvorrichtung nach Anspruch 1, bei der die erste Kammer über zwei Einwegventile mit der Aorta oder der linken Herzkammer verbindbar ist, und die zweite Kammer mit den oberen Atemwegen verbindbar ist, und daher eingerichtet ist, mit Luft mit variablem Druck versorgt zu werden, wobei die erste und die zweite Kammer durch eine elastische Membran derart voneinander getrennt sind, dass durch die Bewegung der elastischen Membran infolge des Atmungszyklus Blut gepumpt wird.

5. Kreislaufunterstützungsvorrichtung nach Anspruch 1, bei der die erste Kammer über zwei Einwegventile mit der Aorta oder der linken Herzkammer verbindbar ist, und die zweite Kammer über einen Schlauch mit der Pleurahöhle verbindbar ist, und daher mit Luft mit variablem Druck versorgbar ist, wobei die erste und die zweite Kammer durch eine elastische Membran derart voneinander getrennt sind, dass durch die Bewegung der elastischen Membran infolge des Atmungszyklus Blut gepumpt wird.

6. Kreislaufunterstützungsvorrichtung nach Anspruch 1, bei der die erste Kammer (2) ohne die Notwendigkeit eines Ventils mit der Aorta (3) oder der linken Herzkammer verbindbar ist, die zweite Kammer über einen Schlauch (8) entweder mit den oberen Atemwegen (7) oder mit der Pleurahöhle verbindbar ist, und eine elastische Membran (1) die erste und die zweite Kammer derart voneinander trennt, dass die elastische Membran durch die Atembewegungen bewegt wird, was ein periodisches Pumpen von Blut in den Kreislauf bewirkt.

7. Kreislaufunterstützungsvorrichtung nach Anspruch 6, bei der die Verbindung zwischen der ersten Kammer (2) und der Aorta oder der linken Herzkammer mittels eines Kanals hergestellt wird.

## Revendications

1. Nouveau dispositif d'assistance circulatoire adapté pour utiliser l'énergie du système respiratoire du patient lui-même, le dispositif d'assistance comprenant une pompe constituée d'une première chambre (2) adaptée pour être raccordée au circuit sanguin (3) et d'une seconde chambre (6) adaptée pour être raccordée au système respiratoire (7), dans lequel le sang circule dans le circuit sanguin lorsque les variations de pression d'air induites pendant le cycle respiratoire provoquent des variations de pression dans la seconde chambre (6).

2. Nouveau dispositif d'assistance circulatoire selon la revendication 1, qui peut fonctionner avec l'énergie dérivée des mouvements musculaires du patient lui-même.

3. Nouveau dispositif d'assistance circulatoire selon la revendication 1, comprenant des ventilateurs mécaniques raccordés au dispositif d'assistance, de sorte que l'assistance circulatoire est favorisée chez les patients intubés avec un choc cardiogénique.

4. Dispositif d'assistance circulatoire selon la revendication 1, dans lequel la première chambre est adaptée pour être raccordée à l'aorte ou au ventricule gauche par le biais de deux valves à une voie et la seconde chambre est adaptée pour être raccordée aux voies respiratoires supérieures, par conséquent étant configurée pour être alimentée par de l'air de pression variable, dans lequel les première et seconde chambres sont séparées par une membrane élastique, de sorte que le mouvement de la membrane élastique dû au cycle respiratoire provoque le pompage du sang.

5. Dispositif d'assistance circulatoire selon la revendication 1, dans lequel la première chambre est adaptée pour être raccordée à l'aorte ou au ventricule gauche par le biais de deux valves à une voie et la seconde chambre est adaptée pour être raccordée à la cavité pleurale par le biais d'un tube, par conséquent étant configurée pour être alimentée par de l'air de pression variable, dans lequel les première et seconde chambres sont séparées par une membrane élastique, de sorte que le mouvement de la membrane élastique dû au cycle respiratoire, provoque le pompage du sang.

6. Dispositif d'assistance circulatoire selon la revendication 1, dans lequel la première chambre (2) est adaptée pour être raccordée à l'aorte (3) ou au ventricule gauche sans avoir besoin de valve, la seconde chambre est adaptée pour être raccordée aux voies respiratoires supérieures (7) ou à la cavité pleurale par le biais d'un tube (8), et une membrane élastique (1) sépare les première et seconde chambres de sorte que la membrane élastique est déplacée par les mouvements respiratoires, amenant ainsi le sang à être pompé de manière périodique dans la circulation.

7. Dispositif d'assistance circulatoire selon la revendication 6, dans lequel le raccordement entre la première chambre (2) et l'aorte ou le ventricule gauche est réalisé au moyen d'un conduit.
